(19)

Europäisches
Patentamt
European
Patent Office
Office européen
des brevets

(11) **EP 1 886 681 A2**

(12) **EUROPEAN PATENT APPLICATION**

(43) Date of publication:
**13.02.2008 Bulletin 2008/07**

(21) Application number: **07022534.7**

(22) Date of filing: **07.10.2004**

(51) Int Cl.:
*A61K 31/385* (2006.01)    *A61K 47/48* (2006.01)
*A61P 9/00* (2006.01)    *A61P 19/00* (2006.01)
*A61P 11/00* (2006.01)    *A61P 1/00* (2006.01)
*A61P 15/00* (2006.01)    *A61P 13/00* (2006.01)
*A61P 25/00* (2006.01)    *A61P 17/00* (2006.01)
*A61P 35/00* (2006.01)    *A61P 31/00* (2006.01)

(84) Designated Contracting States:
**AT BE BG CH CY CZ DE DK EE ES FI FR GB GR
HU IE IT LI LU MC NL PL PT RO SE SI SK TR**

(62) Document number(s) of the earlier application(s) in
accordance with Art. 76 EPC:
**04425751.7 / 1 645 288**

(71) Applicant: **CTG Pharma S.r.l.**
**20131 Milan (IT)**

(72) Inventor: **Sparatore, Anna**
**20146 Milan (IT)**

(74) Representative: **Postiglione, Ferruccio et al
Ufficio Internatzionale Brevetti
Ing. C. Gregorj S.p.A.
Via Dogana 1
20123 Milan (MI) (IT)**

Remarks:
This application was filed on 21-11-2007 as a
divisional application to the application mentioned
under INID code 62.

(54) **New nuclear transcription factors regulators**

(57)    The present invention relates to novel compounds that are nuclear transcription factors (NTF) regulators. The present invention also provides methods for treating, preventing and/or reducing inflammation-associated diseases by regulating NTF in the cardiovascular, connective tissue, pulmonary, gastrointestinal, respiratory, urogenital, nervous, or cutaneous systems as well as tumoral and infective diseases employing said compounds.

EP 1 886 681 A2

**Description**

Field of the invention

This invention relates to the field of compounds able to regulate nuclear transcription factors (NTFs). NTFs activation is involved in a variety of human diseases such as atherosclerosis, asthma, arthritis, cancer, diabetes, AIDS, inflammatory bowel diseases and stroke. The development of NTF regulators should reduce side effects associated with drugs such as, for example, NSAIDs, glucocorticoids etc.

Background of the invention

Many NTFs have been described in the literature. A non exclusive list of factors that are involved in the inflammatory process include nuclear factor kappa B (NF-kB), activator protein 1 (AP-1), specificity protein 1 (Sp1), peroxisome proliferator-activated receptors (PPARs), Nr2/small Maf and other members of the nuclear receptor superfamily (K. Kataoka et al. in J. Biol. Chem. 2001, 276 (36), pag. 34074-81 and Y. Lavrovsky et al. in Exp. Gerontol. 2000, 35, pag. 521-32).

Among these NTFs, NF-kB is one of the most studied and will be described as non-limitative example. It was discovered by David Baltimore's group in 1986, ubiquitously expressed that represents a group of five mammalian, structurally related, proteins also called Rel/NF-kB proteins (R. Sen and D. Baltimore in Cell 1986, 47, pag. 921-928). NF-kB plays a central role in regulating expression of a large number of genes that are critical for the regulation of apoptosis, viral replication, tumorigenesis, inflammation etc. Cytokines that are stimulated NF-kB, such as IL-1$\beta$ and TNF-$\alpha$, can also directly activate the NF-kB pathway, thus establishing loops that can amplify the inflammatory response and increase the duration of chronic inflammation. NF-kB also stimulates the expression of enzymes whose products contribute to the pathogenesis of inflammatory process, including the inducible form of nitric oxide synthase (iNOS), which generates nitric oxide (NO), and the inducible cyclooxygenase (COX2), which generates prostanoids (Y. Yamamoto and R. Gaynor in J. Clin. Invest. 2001, 107, pag. 135-142). The activation of NF-kB is thought to be part of a stress response as it is activated by various factors that include growth factor, cytokines, lymphokynes, viral proteins, ischemia/ reperfusion, UV, pharmacological agents, and oxidative. Generally, inflammation has been implicated in a very large number of disorders either as a cause of primary disease process or at least as consequence leading to mild/severe complications. Therefore, there is a need in the art for more effective and safer drugs for inflammation-associated disorders in the cardiovascular system (for example myocardial and vascular ischemia in general, hypertension systemic and regional, stroke, atherosclerosis, etc), connective tissue (for example arthritis and connected inflammatory diseases, etc.), pulmonary ssstem (for example asthma, COPD, etc.), gastrointestinal system (for example ulcerative and non-ulcerative diseases, intestinal inflammatory diseases, liver cirrhosis, etc), urogenital system (for example impotence, incontinence, etc.), central nervous system (Alzheimer disease, Parkinson's disease and neurogenerative diseases in general), cutaneous system (eczema, neurodermatitis, acne, etc.), infective disease (of bacteria, viruses, parasites origin) and for antineoplastic therapy in different organs (colon, lung, prostate, ovaries, uterus, breast, tongue, liver, bone, etc.), in prevention and/or treatment as single therapy or cotherapy with other chemiotherapic agents or radiotherapic interventions.

Tissue damage by infection, trauma, toxins, abnormally low or high temperatures and other reasons, usually leads to formation of increased amounts of pathogenetic mediators, such as prostaglandins, cytokines, leukotrienes, interleukins, oxy-, thiyl- and nitrogen-free radicals, interacting each other. A serious, outstanding medical need is the development of NTF specific inhibitors that afford an efficacious treatment while minimizing unwanted effects. The research has been focused in the past to develop effective therapeutic agents able to counteract the deleterious effects of such mediators, but results have been so far either unsatisfactory or only partially satisfactory. In fact it has been described (see for example the review by Tak PP and Firestein GS JCI,107,7-11,2001) that some compounds, including corticosteroids, sulfasalazine, 5-aminosalicylic acid, aspirin, NO-releasing aspirin, tepoxalin, leflunomide, curcumin, antioxidants, proteasome inhibitors are able to inhibit NF-kB only at relatively high concentrations. Thus this appears to be the cause for the preclusion of the utilization of NF-kB inhibitors in therapy. An alternative and/or complementary approach is to develop agents able to potentiate the natural defences. In this class of agents a variety of moieties have been described, including again in a non-limitative and exclusive way: heme oxygenase-1, y-glutamylcysteine synthetase, IL10, Mn-SOD, Catalase, DT-diaphorase, glutathione peroxidase, thioredoxin system, NADPH-oxidase, NAG-1, p53.

The ideal approach is to develop compounds able to both counteract aggressive and pathogenetic factors and potentiate defensive substances. This can be obtained by combining chemically a drug known to counteract one or more of the above mentioned pathogenetic mediators with other moieties able to regulate the activation of NTF.

The new compounds object of the present invention, that are able to inhibit the formation of a pathogenetic mediator, such as inducible NOS at non-toxic concentrations, and to stimulate the formation of a defensive agent, such as heme oxygenase-1, have the following chemical structure:

D-X-Y-S          (I)

wherein D is a drug, X is zero or a chemical functional group, such as C=O, COOR, CONH, R being straight or branched $C_1$-$C_4$-alkyl,
Y is zero or a bifuntional linker, such as HO-$(CH_2)$n-OH, HOOC-$(CH_2)_n$-COOH, and S is a moiety capable per se or in combination with the drug to modulate the NTFs, with the proviso that when both X and Y are zero, the drug (D) and the moiety (S) are linked by an acid-base bond

$$D^{+/-} \cdot S^{-/+}$$

wherein D is an acid (+) or basic (-) drug and S is a basic (-) or acid (+) compound capable to modulate directly and/or indirectly the NTF that is
a salt of an acid (drug$^-$ ) with a base (NFs$^+$ modulator) or a salt of a base (drug$^+$) with an acid (NFs- modulator).

## General

For the screening approach it was decided to test the compounds object of the present invention and fulfilling the structural general criteria previously described for heme oxygenase-1 (OH-1)and inducible nitric oxide synthase (iNOS). The enzyme tests were performed as described by Vicente AM et al. (Br.J.Pharmacol. 2001,133,920-6). The formation of both these two enzymes was associated with the activation of transcription factors, and NF-kB particularly. The following reference compounds were used: indomethacin as example of unselective cyclooxygenase inhibitor, dexamethasone, as example of steroidal glucorticoid agent, N-acetylcysteine as example of anti-oxidant agent, NS-398 as example of COX-2 inhibitor, zileuton, as example of 5-lipooxygenase inhibitor,L-N-methylarginine (L-NAME), as example of NOS inhibitor.
In brief, Raw 264.7 murine macrophage cell line were maintained in a proper culture medium supplemented with 10% fetal bovine serum,2 mM L-glutamine and penicillin/streptomycin. Suspensions of zymosan A from Saccharomyces cerevisiae in saline were used. After incubation with zymosan and/or test compounds for 18 hrs, cells supernatants were collected to measure protein expression, by western blot analysis. After incubation, macrophages were lysed in 100 microliters of buffer (1% Triton X-100,1% deoxicholic acid,20 mM NaCl and 25 mM Tris,pH 7.4) and then centrifuged at 4°C for 5 min at 10,000 x g. The protein content was determined by the Bradford method using bovine serum albumin as standard. Cell lysate (40 micrograms of protein) was mixed with Laemmli sample buffer under reducing conditions. Samples were sized-separated in 12.5% SDS-PAGE and transferred into polyvinylidene membranes, which were blocked in phosphate buffer saline (0.02 M,pH 7.0)-Tween 20 (0.1%) containing 3% fat-free dry milk. Membranes were incubated with specific antibodies: polyclonal antibody against iNOS (1/1000,Cayman Chemical) and anti-OH-1 monoclonal antibody (1/2000,Stressgen). Blots were washed and incubated with peroxidase-conjugated goat IgG (1/20,000). The immunoreactive bands were visualized by an enhanced chemiluminescence system and band intensity quantitated using computer-assisted densitometry.
Cell viability was assessed by the mitochondrial-dependent reduction of 3-(4,5-dimethyltiazol-2-yl)-2.5 diphenyltetrazolium bromide (MTT) to formazan. After appropriate stimulation times, cells were incubated with MTT (200 micrograms/ml) for 60 min. The medium was then removed and cells were solubilized in dimethylsulphoxide to quantitate formazan at 550 nm.

## Results

The results on OH-1 and iNOS were shown in table I. Only the test compound ATANPE was able to stimulate OH-1 production and inhibit iNOS formation. All the compounds did not affect cell integrity and viability under the experimental conditions above described and at the concentrations used.

Table I

| Effect of some compounds on zymosan-induced OH-1 and iNOS protein expression. Band intensity was calculated as percentage with respect to zymosan. | | | |
|---|---|---|---|
| Treatment | Concentration ($\mu$mol) | OH-1 | INOS |
| Basal | - - - - - - | 3 | 2 |

(continued)

| Effect of some compounds on zymosan-induced OH-1 and iNOS protein expression. Band intensity was calculated as percentage with respect to zymosan. | | | |
| --- | --- | --- | --- |
| Treatment | Concentration ($\mu$mol) | OH-1 | INOS |
| Zymosan | - - - - - - | 100 | 100 |
| Dexamethasone | 1 | 20 | 85 |
| Indomethacin | 1 | 65 | 78 |
| NS398 | 10 | 72 | 120 |
| N-acetylcysteine | 100 | 44 | 80 |
| Zileuton | 10 | 125 | 112 |
| L-NAME | 1000 | 70 | 110 |
| ATANPE | 10 | 293 | 48 |

It has been found that the compounds fulfilling these criteria surprisingly showed not only an efficacy greatly increased, with additive or synergistic implementation, but also a safety profile dramatically improved.

Summary of the invention

The present invention is based on the discovery that it is possible to link regulators of NTF to a pharmacologically active compound helpful for treating disorders in the cardiovascular, connective tissue, pulmonary, gastrointestinal, respiratory, urogenital, nervous, or cutaneous systems or tumoral and infective diseases, i.e. compounds for treating, preventing or reducing oxidative stress associated with cardiovascular, respiratory, connective tissue, nervous, gastrointestinal, tumoral, cutaneous, infective, urogenital diseases. The resulting compounds have good bioavailability, increased activity and/or safety.

The parent drugs (i.e. the drugs in which the modification with NF-regulating moiety can be applied) in the present invention can be selected within a wide class of compounds, such as NSAIDs (i.e. unselective cyclo-oxygenase (COX) inhibitors, - ketoprofen, flurbiprofen, suprofen, indobufen, etodolac, indomethacin, naproxen etc.), analgesics (i.e. paracetamol, capsaicin), steroids (chenodeoxycholic acid, flunisolide), antibiotics (bacilysin), bronchodilators (albuterol), expectorants and mucolitic agents (ambroxol), antiasthma, antiallergic and anti-histaminic drugs (epinastine), ACE-inhibitors (captopril), $\beta$-blockers (atenolol), drugs for vascular disorders (brovincamine), antidiabetics (glibomuride), antitumorals (ancitabine, mopidamol), antiulcer (arbaprostil), antihyperlipidemics (fluvastatine), antibacterials (amoxicilline), antivirals (amantadine), cGMP phosphodiesterase inhibitors (sildenafil, verdenafil), drugs against dementia (mofegiline) and the bone reabsorption (alendronic acid). Also compounds that are nitric oxide donors are useful in the present invention.

When the compounds include at least one asymmetric carbon atom, the products can be used in racemic mixture or in form of single enantiomer.

In the present invention the parent compound is considered in its original form or in a proper modification to allow the chemical manipulation with NF-regulating moieties.

Also parent drugs able to release nitric oxide exogenously or endogenously are useful for the present invention.

NF-regulating moieties include $\alpha$-lipoic acid, $\alpha$-tocoferol, butylated hydroxyanisole (BHA), caffeic acid derivatives, catechol derivatives, cinnamic acid, curcumin, epigallocatechin-3-gallate, ergothioneine, N-(p-aminobenzoyl) glutamic acid, glutathione, hematein, magnolol, nordihydroguaiaretic acid (NDGA), phenolic antioxidants, pyrrolidine dithiocarbamate (PDTC), quercetin, resveratrol, Vitamin C, vitamin E, salicylic acid derivatives (such as cresotic acid isomers and 4-hydroxyisophthalic acid).

Other substances releasing or stimulating the release of hydrogen sulfide and NF-regulating that can be linked to drugs are N-acetyl-penicillamine, S-allyl-cysteine, bucillamine, carbocysteine, cysteamine, cystathionine, homocysteine, mecysteine, methionine, pantetheine, penicillamine, penicillamine disulfide, thioacetic acid, thiodiglycolic acid, thioglycolic acid, thiolactic acid, 2-thiolhistidine, thiomalic acid, thiosalicylic acid, tiopronin. Other substances releasing and/or stimulating the release of hydrogen sulfide and NF-regulating that can be linked to drugs are 5-(p-hydroxyphenyl)-3H-1,2-dithiol-3-thione, 1,3 dithiol-2-thione-5-carboxylic acid, 3-thioxo-3H-1,2-dithiole-5-carboxylic acid, 3-thioxo-3H-1,2-dithiole-4 carboxylic acid.

More groups NF-regulating by releasing $H_2S$ also in combination with groups that release nitric oxide or carbon oxide or that release nitric oxide or carbon oxide per se are part of the present invention.

The substances can be linked via different linking groups such as esters, amides, imides, sulfonamides, azo groups, carbamates, carbonates, anhydrides, acetals, thioacetals, etc.

Bifunctional linkers known to the expert in the field (such as ethyl, propyl, or butyl diols; diamines; hydroxy amines, etc..) can be optionally present when they are necessary to link the drug to the NF-regulating moieties.

Also salts that directly or indirectly are capable to inhibits NF-kB, such as, for example, salts of arginine, agmatine, aminoguanidine, penicillamine, lipoic acid, caffeic acid, 1,3 dithiol-2-thione-5-carboxylic acid, thiosalicylic acid, cinnamic acid etc. are part of the present invention.

The copounds of the present invention are valproic acid ester with 5-(p-hydroxyphenyl)-3H-1,2-dithiol-3-thione and also the salcylic acid derivatives which are cresotic acid isomers and 4-hydroxyisophthalic acid, ketoprofen ester with 5-(p-hydroxyphenyl)-3H-1,2-dithiol-3-thione, ketorolac ester with 5-(p-hydroxyphenyl)-3H-1,2-dithiol-3-thione, acetylthiosalicylic acid 4-(nitroxymethyl)phenyl ester.

The compounds of the present invention can be administered in the form of any pharmaceutical formulation, the nature of which will depend upon the route of administration and the nature of the disease to be treated. These pharmaceutical compositions can be prepared by conventional methods, using compatible, pharmaceutically acceptable excipients or vehicles. Examples of such compositions include capsules, tablets, syrups, powders and granulates for the preparation of extemporaneous solutions, injectable preparations, rectal, nasal, ocular, vaginal etc. A preferred route of administration is the oral route.

The following non-limitative examples further describe and enable an ordinary skilled in the art to make and use the invention.

### EXAMPLE 1. Synthesis of 5-(p-hydroxyphenyl)-3H-1,2-dithiol-3-thione.

To 280 mmol of sulfur 40 mmol of anethole were added. After heating at 200 °C for 6 hours, 2.5 g of anethole dithiolethione were obtained. The product, washed with ether, was crystallized by ethyl acetate: melting point 110-111°C. Then 1.5 g of anethole dithiolethione were mixed with 7.5 g of pyridine HCl and the mixture was heated for 25 minutes at 215°C. After cooling, 1N HCl in excess was added and the precipitate was filtered, washed and crystallized from ethanol. The obtained compound melted at 191-192°C.

### EXAMPLE 2. Synthesis of valproic acid ester with 5-(p-hydroxyphenyl)-3H-1,2-dithiol-3-thione.

The ester of valproic acid with 5-(p-hydroxyphenyl)-3H-1,2-dithiol-3-thione was prepared via the acyl chloride of valproic acid. The acyl chloride of valproic acid (3.0 mmol) and the compound prepared in the example 1 (1.59 mmol) were refluxed in THF anhydrous for 6 hours under nitrogen atmosphere. After removal of THF, the mixture was chromatographed on silica gel eluting with dichloromethane. The compound was crystallized from ethyl ether and showed a melting point of 69.5-70.5°C. Anal. calcd. $C_{17}H_{20}O_2S_3$ C% 57.92; H% 5.72; S% 27.29; found C% 58.05; H% 5.69; S% 27.34.

### EXAMPLE 3. Synthesis of ketoprofen ester with 5-(p-hydroxyphenyl)-3H-1,2-dithiol-3-thione.

The ketoprofen ester was prepared via its acyl chloride and 5-(p-hydroxyphenyl)-3H-1,2-dithiol-3-thione. 2.3 mmol of ketoprofen were suspended in 7 ml of anhydrous THF with 0.92 ml of thionyl chloride, heating at 67°C for 6 hours. After evaporation of THF and thionyl chloride, the ketoprofen acyl chloride was dissolved in anhydrous THF, and a solution of 5-(p-hydroxyphenyl)-3H-1,2-dithiol-3-thione (1.77 mmol.), pyridine (0.2 ml) in THF anhydrous (1 ml) was dropped therein, and the reaction was kept under stirring overnight at room temperature. After evaporation of THF, the product thus obtained was chromatographed on silica gel eluting with dichloromethane. The purified product had a gummy glue-like appearance and its crystallization was obtained adding few drops of ether to the refrigerated compound. The orange crystals showed a melting point of 111.5 - 112.5°C. Anal. calcd. $C_{25}H_{18}O_3S_3$ C% 64.91; H% 3.92; S% 20.79; found C% 64.83; H% 4.02; S% 20.49.

### EXAMPLE 4. Synthesis of ketorolac ester with 5-(p-hydroxyphenyl)-3H-1,2-dithiol-3-thione.

The ketorolac ester was prepared employing dicyclohexylcarbodiimide (DCC) as coupling agent in presence of 4-pyrrolidinylpyridine.
The solvent used was methylene chloride purified on basic $Al_2O_3$ to remove traces of ethanol. In a 50 ml flask, 1.17 mmol of ketorolac and 2.15 ml of methylene chloride were charged as well as 0.014 mol of 4-pyrrolidinylpyridine, 1.17 mmol of 5-(p-hydroxyphenyl)-3H-1,2-dithiol-3-thione and 1.47 mmol of DCC (25% excess) in 1.5 ml of methylene chloride. The mixture was stirred for 30 minutes at room temperature.
At the end of the reaction, after filtration the solution was extracted with 10% acetic acid, then with 0.1N NaOH and finally with brine. After removal of the solvent, the product was chromatographed on silica gel with dichoromethane and crystallized by ether: melting point 146-147°C. Anal. calcd. $C_{24}H_{17}NO_3S_3$ C% 62.18; H% 3.69; N% 3.02; S% 20.75; found C% 61.85; H% 3.60; N% 2.83; S% 21.10.

## EXAMPLE 5. Synthesis of acetyl thiosalicylic acid 4-(nitroxymethyl)phenyl ester (ATANPE).

To a solution of thiosalicylic acid (39.9 mmol) in KOH (5.6 g in 56 ml of water), 48.9 mmol of acetic anhydride were added under stirring for 1 hour on a ice bath.
Adding 4N HCl a white precipitate was obtained. The solid was filtered and dried.

To a suspension of 5 mmol of the solid in 5 ml of chloroform, 10.3 mmol of oxalyl chloride were added. The mixture was heated at 86 °C and at the end of the reaction the chloroform and oxalyl chloride were evaporated under reduced pressure.

To the acyl chloride solubilized in chloroform, 4.17 mmol of p-hydroxybenzaldehyde and 5 mmol of triethylamine were added. The temperature of the reaction was maintained at 0 °C. The acetyl thiosalicylic p-hydroxybenzaldheide ester was chromatographed on silica gel eluting with cycloexane/ethylacetate (8/2). After crystallization with ether, the compound showed a melting point of 93-94 °C. The ester (1.32 mmol) was transformed in the corresponding p-hydroxybenzyl alcohol with sodium triacetoxy borohydride (5.3 mmoles) in dichloroethane heating under reflux.

The last step was the nitration of the alcohol previously prepared. The mixture for the nitration was constituted by 0.5 ml of fuming $HNO_3$, 1.25 ml of acetic anhydride and 10.75 ml of methylene choride. This mixture (2.7 ml) was added to the alcohol derivative (2.7 mmol in 5 ml of dichloromethane) and the reaction was maintained at 0 °C for 1 hour. After dilution with dichloromethane, the solution was extracted with iced water.
The organic phase, dried on anhydrous sodium sulphate, was evaporated. The oily compound, after purification on silica column eluting with dichloromethane and crystallization by ether, had a melting point of 65-66 °C.

## EXAMPLE 6 - Pharmacological

The NF-kB-CAT reporter gene cell line (Lenardo MJ et al.,Cell 58,227-9,1989) and. L29/Ji6 cells (Fratelli M et al, Antioxidants & Redox Signalling 5,229-235,2003) were prepared as previously described. Human TNF alfa (100 ng/ml) was used. The chloramphenicol-acetyltransferase (CAT) activity was measured by adding 40 microliters of Tris-HCl buffer (250 mM, ph 7.5) and D-threo-[dichloroacetyl] 1,2 $^{14}$C chloramphenicol (10 microliters,8.3 x $10^{-2}$ mCi/sample) The reaction was started by the addition of n-butyryl coenzyme A. After 2 h at 37°C, the reaction was stopped by extraction with hexane/xylene (2:1). The upper organic phase containing the reaction product was then recovered, and radioactivity was counted on a beta-counter. The activity on NF-kB was expressed as % of CAT formation.

Table II

| Effect on NF-kB activity by test compound (100 $\mu$M), as assessed by a reporter gene synthesis: % of CAT syntesis | | |
| --- | --- | --- |
| Compound | Basal | TNF-stimulated |
| Aspirin | 100 | 92 |
| ATANPE | 31 | 26 |

## EXAMPLE 7. Inhibition of the expression of NF-kB regulated cytokine IL-6 and IL-8 in HeLa cells by compound described in example 5.

The cells were stimulated with 100 $\mu$mol of compound described in example 6 with TNF (200 E/ml) or with PMA (50ng/ml)and the concentration of IL-6 or IL-8 was measured in the cell supernatant at various interval using ELISA (British Biotechnology Products Ltd.). The expression values are listed in the following table III.

TABLE III

| | IL-6 (pg/ml) | | | IL-8 (pg/ml) | | |
| --- | --- | --- | --- | --- | --- | --- |
| | 0' | 15' | 120' | 0' | 15' | 120' |
| TNF | -- | 945 | 7640 | -- | -- | 61 |
| TNF+EX 5 | -- | 945 | 1738 | -- | -- | 6 |
| PMA | -- | 870 | 11813 | -- | -- | 445 |
| PMA+EX 5 | -- | 920 | 3138 | -- | -- | 12 |

The values show that after 120 minutes the compound described in example 5 is able to inhibit the production of IL-6 by 87% or 74% (TNF stimulation) and the production of IL-8 by 90% or 97% (PMA stimulation).

**Claims**

1. Valproic acid ester with 5-(p-hydroxyphenyl)-3H-1,2-dithiol-3-thione.

2. Compound according to claim 1 for use as a medicament.

3. Compound according to claim 1 for treating, preventing or reducing oxidative stress associated with cardiovascular, respiratory, connective tissue, nervous, gastrointestinal, tumoral, cutaneous, infective, urogenital diseases.

4. Compound according to claim 1 for treating, preventing or reducing tumoral diseases as single or with other chemiotherapic agents.

5. Compound according to claim 1 for treating, preventing or reducing tumoral diseases in different organs (colon, lung, prostate, ovaries, uterus, breast, tongue, liver, bone), as single or with other chemiotherapic agents.

6. Compound according to claim 1 for treating, preventing or reducing tumoral diseases with radiotherapic interventions.

7. Compound according to claim 1 for treating, preventing or reducing tumoral diseases in different organs (colon, lung, prostate, ovaries, uterus, breast, tongue, liver, bone), with radiotherapic interventions.

8. Use of compound according to claim 1 for the manufacturing of a medicament for treating, preventing or reducing oxidative stress associated with cardiovascular, respiratory, connective tissue, nervous, gastrointestinal, tumoral, cutaneous, infective, urogenital diseases.

9. Use of compound according to claim 1 for the manufacturing of a medicament for treating, preventing or reducing tumoral diseases as single or with other chemiotherapic agents.

10. Use of compound according to claim 1 for the manufacturing of a medicament for treating, preventing or reducing tumoral diseases in different organs (colon, lung, prostate, ovaries, uterus, breast, tongue, liver, bone), as single or with other chemiotherapic agents.

11. Use of compound according to claim 1 for the manufacturing of a medicament for treating, preventing or reducing tumoral diseases with radiotherapic interventions.

12. Use of compound according to claim 1 for the manufacturing of a medicament for treating, preventing or reducing tumoral diseases in different organs (colon, lung, prostate, ovaries, uterus, breast, tongue, liver, bone), with radiotherapic interventions.

13. Pharmaceutical composition comprising compound according to claim 1 as active ingredient and a pharmaceutically acceptable adjuvant or carrier.

14. Pharmaceutical composition comprising compound according to claim 1 for for treating, preventing or reducing oxidative stress associated with cardiovascular, respiratory, connective tissue, nervous, gastrointestinal, tumoral, cutaneous, infective, urogenital diseases.

# EP 1 886 681 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **K. KATAOKA et al.** *J. Biol. Chem.,* 2001, vol. 276 (36), 34074-81
- **Y. LAVROVSKY et al.** *Exp. Gerontol.,* 2000, vol. 35, 521-32
- **R. SEN ; D. BALTIMORE.** *Cell,* 1986, vol. 47, 921-928
- **Y. YAMAMOTO ; R. GAYNOR.** *J. Clin. Invest.,* 2001, vol. 107, 135-142
- **VICENTE AM et al.** *Br.J.Pharmacol,* 2001, vol. 133, 920-6
- **LENARDO MJ et al.** *Cell,* 1989, vol. 58, 227-9